Europäisches Patentamt

European Patent Office

Office européen des brevets

(11) Veröffentlichungsnummer: **0 335 067 B1**

# (12) EUROPÄISCHE PATENTSCHRIFT

(45) Veröffentlichungstag der Patentschrift: **17.03.93**

(51) Int. Cl.5: **C07C 209/06**, C07C 211/63

(21) Anmeldenummer: **89100694.2**

(22) Anmeldetag: **17.01.89**

(54) Verfahren zur Herstellung von Tetrapropylammoniumbromid.

(30) Priorität: **31.03.88 DE 3810953**

(43) Veröffentlichungstag der Anmeldung:
**04.10.89 Patentblatt 89/40**

(45) Bekanntmachung des Hinweises auf die Patenterteilung:
**17.03.93 Patentblatt 93/11**

(84) Benannte Vertragsstaaten:
**AT BE DE FR GB NL SE**

(56) Entgegenhaltungen:
EP-A- 0 104 107
US-A- 3 965 178

CHEMICAL ABSTRACTS, Band 108, Nr. 19, 9. Mai 1988, Seite 598, Spalte 1, Zusammenfassung Nr. 166930b, Columbus, Ohio, USA; SHEN YUSHENG et al: "Aggregation of ion pairs and synthesis of six cymmetrical tetraalkylammonium bromide salts. IV"

PETROCHEMIA, Band 23, 1983, Nr. 2-3, Seite 69-72; D. MRAVEC et al: "Kinetika kvarternizacie tri-n-propylaminu n-propylbromidom A n-propyljodidom"

BERICHTE DER DEUTSCHEN CHEMISCHEN GESELLSCHAFT, 35. Jahrgang, 1902, P. JACOBSON: "Das Tetrapropylammoniumjodid"

(73) Patentinhaber: **Degussa Aktiengesellschaft**
**Weissfrauenstrasse 9**
**W-6000 Frankfurt am Main 1(DE)**

(72) Erfinder: **Siray, Mustafa, Dr.**
**Gleiwitzer Strasse 3**
**W-6450 Hanau 9(DE)**
Erfinder: **Kleinschmit, Peter, Dr.**
**Wildaustrasse 19**
**W-6450 Hanau 9(DE)**

**Beschreibung**

Die Erfindung betrifft ein Verfahren zur Herstellung von Tetrapropylammoniumbromid.

Tetrapropylammoniumbromid wird als Reagenz bei der Herstellung von Zeolith ZSM-5 eingesetzt (US-PS 3,702,886 und EP-PS 41 621).

Aus der EP-PS 41 621, Seite 1, Zeile 15 ist bekannt, daß Tetrapropylammoniumbromid eine nur schwer zugängliche quarternäre Stickstoffbase ist.

Das Dokument Petrochemia, Band 23, 1983, Nr.2 bis 3, Seite 69 bis 72 beschreibt die Umsetzung von Tri-n-propylamin mit n-Propylbromid in Ethanol bei einer Temperatur von 35 bis 65°C.

Es besteht somit ein Bedürfnis nach einem einfachen und wirtschaftlichen Verfahren zur Herstellung von Tetrapropylammoniumbromid.

Gegenstand der Erfindung ist ein Verfahren zur Herstellung von Tetrapropylammoniumbromid, welches dadurch gekennzeichnet ist, daß man Tripropylamin mit Propylbromid in einem polaren Lösungsmittel aus der Gruppe Nitromethan, Hexamethylphosphorsäuretriamid, Dimethylformamid oder N,N-Dimethylpyrol bei einer Temperatur von 80 bis 160 °C umsetzt, wobei man ein Verhältnis von Tripropylamin und Propylbromid zu Lösungsmittel von 20 bis 60 % einhält.

Die Zeit, in der die Umsetzung durchgeführt wird, kann 2 bis 32, vorzugsweise 2 bis 15 oder 4 bis 12 Stunden betragen.

Die Reaktionstemperatur kann vorzugsweise 80 bis 140 °C, insbesonders 100 bis 130 °C betragen.

Das Verhältnis von Tripropylamin und Propylbromid zu Lösungsmittel 20 bis 60 % beträgt. Dieser Wert errechnet sich aus der Formel

$$\frac{\text{ml Lösungsmittel}}{\text{ml Propylbromid} + \text{ml Tripropylamin}} \cdot 100 = \text{Verhältnis}$$

Das erfindungsgemäße Verfahren hat den Vorteil, daß bei dem Abkühlen des Lösungsmittels bzw. der Reaktionsmischung 65 Gew.-% des Produktes analysenrein und kristallin ausfällt (bezogen auf Tripropylamin).

Die Mutterlauge kann ohne Aufarbeiten für den nächsten Ansatz verwendet werden.

Ein Umkristallisieren des Reaktionsproduktes ist nicht notwendig.

Beispiel 1

135 g (1,09 mol) Propylbromid und 140 g (0,96 mol) Tripropylamin werden mit 200 ml Dimethylformamid in einer mit Rückflußkühler und Thermometer versehene Apparatur 6 Stunden unter Rühren bis 130 °C erwärmt. Der Endpunkt ist am Verschwinden der zwei Phasen, die sich aus Propylbromid und Dimethylformamid/Tripopylamin bilden, und Temperaturanstieg erkennbar. Beim Erkalten der so hergestellten Lösung fallen Tetrapropylammoniumbromid-Kristalle aus. Sie werden abfiltriert und getrocknet. Ausbeute: 162 g (63 % bezogen auf Tripropylamin)

Beispiel 2

135 g (1,09 mol) Propylbromid und 140 g (0,96 mol) Tripropylbromid werden mit der Mutterlauge aus Beispiel 1 zusammengegeben und erneut 6 Stunden bis 130 °C erwärmt. Beim Abkühlen der Lösung fallen Tetrapropylammoniumbromid-Kristalle aus. Ausbeute: 174 g (65 % bezogen auf Tripropylamin)

Diese Mutterlauge kann erneut eingesetzt werden. Durch Einengen auf die Hälfte des Volumens können aus der Mutterlauge noch weitere 50,8 g (19 % bezogen auf Tripropylamin) Tetrapropylammoniumbromid isoliert werden.

Beispiel 3

Versuch analog zu Beispiel 1, jedoch mit 50 ml Dimethylformamid durchgeführt. Ausbeute: 131 g (49 % bezogen auf Tripropylamin).

Beispiel 4

Versuch analog zu Beispiel 1, jedoch mit 600 ml Dimethylformamid durchgeführt. Ausbeute: 115 g (43 % bezogen auf Tripropylamin)

Beispiel 5

125 g (1 mol) Propylbromid und 140 g (0,96 mol) Tripropylbromid werden mit 200 ml Nitromethan 12 Stunden unter Rühren bei 50 °C erwärmt. Beim Abkühlen der so hergestellten Lösung fallen Tatrapropylammoniumbromid -Kristalle aus. Sie werden abfiltriert und getrocknet. Ausbeute: 121 g (47,5 % bezogen auf Tripropylamin)
Durch Einengen der Mutterlauge können noch weitere 92 g (36 % bezogen auf Tripropylamin) Tetrapropylammoniumbromid isoliert werden.

Verwendungsbeispiel

643 g Wasserglas (375,6 g/l $SiO_2$, 109,8 g/l $Na_2O$) werden in 2 l Wasser gelöst. Zu dieser Lösung gibt man unter Rühren nacheinander 14,5 g Natriumaluminat, gelöst in 500 ml Wasser, 41,5 g Schwefelsäure (96 Gew.-%), gelöst in 500 ml Wasser und 33 g Tetrapropylammoniumbromid aus Beispiel 1, gelöst in 100 ml Wasser zu.
Dieses Gel wird anschließend in einem Stahlautoklaven gefüllt und bei 150 °C 78 Stunden kristallisiert. Nach beendeter Kristallisation wird das Produkt abfiltriert, gewaschen, bei 120 °C getrocknet und calciniert (500 °C /24 h). Dieses Alumosilikat ist ein gut kristallisiertes Produkt, das die Röntgenbeugungslinien des bekannten Zeolithen ZSM-5 aufweist.

**Patentansprüche**

1. Verfahren zur Herstellung von Tetrapropylammoniumbromid, dadurch gekennzeichnet, daß man Tripropylamin mit Propylbromid in einem polaren Lösungsmittel aus der Gruppe Nitromethan, Hexamethylphosphorsäuretriamid, Dimethylformamid oder N,N-Dimethypyrol bei einer Temperatur von 80 bis 160 °C umsetzt, wobei man ein Verhältnis von Tripropylamin und Propylbromid zu Lösungsmittel von 20 bis 60 % einhält.

**Claims**

1. A process for the preparation of tetrapropyl ammonium bromide, characterised in that tripropylamine is reacted with propyl bromide in a polar solvent selected from nitromethane, hexamethylphosphoric acid triamide, dimethylformamide and N,N-dimethylpyrrole at a temperature of from 80 to 160°C, the ratio of tripropylamine and propyl bromide to solvent being maintained at 20 to 60%.

**Revendications**

1. Procédé de fabrication de bromure de tétrapropylammonium, caractérisé en ce que :
   - on fait réagir de l'amine tripropylique avec du bromure propylique dans un solvant polaire appartenant au groupe nitrométhane, triamide d'acide phosphorique hexaméthylique, diméthylformamide ou N,N-diméthylpyrol à une température de 80 à 160°C, tout en maintenant un rapport entre l'amine tripropylique et le bromure propylique par rapport au solvant allant de 20 à 60 %.